# EUROPEAN PATENT APPLICATION

(11) **EP 4 606 905 A1**
(43) Date of publication of application: **27.08.2025**
(21) Application number: 24158877.1
(22) Date of filing: 21.02.2024
(51) Int. Cl.: C12P 21/02, C12M 1/12, C12M 1/00, C12N 7/00

(54) **CONTINUOUS PROTEIN EXPRESSION**

(71) Applicant: Invitris GmbH, 81476 Munich (DE)
(72) Inventor: VOGELE, Kilian, 82152 Planegg-Martinsried (DE); FRANK, Thomas, 82152 Planegg-Martinsried (DE)
(74) Representative: Weickmann & Weickmann PartmbB

(57) **Abstract**

Bacteriophages are viruses that specifically infect a host bacterium and proliferate at the expense of this host. They are composed of proteins that encapsulate a DNA or RNA molecule. Within their lytic state, Bacteriophages replicate within a host bacterium by injecting their viral genetic material into the host cell effectively taking over the cells functions for the production of progeny bacteriophage which leads to the rupture of the cell wall and subsequent bacterial cell death.

## Description

Bacteriophages are viruses that specifically infect a host bacterium and proliferate at the expense of this host. They are composed of proteins that encapsulate a DNA or RNA molecule. Within their lytic state, Bacteriophages replicate within a host bacterium by injecting their viral genetic material into the host cell effectively taking over the cells functions for the production of progeny bacteriophage which leads to the rupture of the cell wall and subsequent bacterial cell death.

The biotechnological applications of bacteriophages are very broad and extend from evolution-based selection methods, like evolutionary improvement of activity of enzymes and other proteins, to phage display by which biological drug substances, e.g. therapeutic antibodies, could be generated and optimized, as well as the application of the bacteriophages themselves as, for example, a substitute for antibiotics.

The later use is based on the natural characteristics of bacteriophages to specifically affect bacteria, in particular pathogenic bacteria, and kill them, usually by lysing the bacteria or by inhibiting the replication system of the bacteria.

This approach that has been applied for a longer time to fight microbial infections and gets more and more attention as the number and spread of multi-drug resistant bacteria strains increases strongly worldwide. Methicillin-resistant Staphylococcus aureus (MRSA) bacteria, for example, is an increasingly common form of infection, often acquired through transmission in hospitals. MRSA infections are extremely difficult to treat using conventional antibiotics. The development of novel antibiotics is significantly slower than this development.

The development of phage-based therapeutics and diagnostics, however, faces challenges related to the production and/or modification of bacteriophages. It has proven difficult to produce the quantities of bacteriophages or modified bacteriophages necessary for therapeutic use.

In this context, cell-free protein production or cell-free protein synthesis (CFPS) using a single-compartment system appears to be limited, both in terms of protein yield and the efficient modification and purification of products.

The present invention addresses these limitations by introducing a new continuous cell-free expression method. Additionally, a system adapted for this novel expression method is provided, along with suitable applications for products produced by this innovative approach.

According to the present invention the new CFPS method is a continuous expression method for the production of at least one protein or multiple proteins, preferably for the production of multi-protein complexes, such as viruses or bacteriophages, in particularly for the production of synthetic viruses or synthetic bacteriophages and wherein translation of the proteins occurs in a liquid-filled reaction space comprising the nucleic acid template and a reaction mix comprising the cell lysate or cell-extract.

In contrast to the prior art, the method of the invention provides a fluid-filled reaction space comprising an inner compartment comprising an inner fluid and an outer compartment comprising an outer fluid, whereby translation occurs in the inner compartment comprising the inner fluid comprising the cell-lysate or cell-extract and the template nucleic acid, and wherein assembly, modification and/or purification of the natural or synthetic protein optionally takes place in the outer compartment comprising the outer fluid.

Hence, a first aspect of the invention relates to a method for continuous expression of at least one protein in a cell free expression system comprising:
(a) providing a fluid-filled reaction space
   comprising an inner compartment comprising an inner fluid and
   an outer compartment comprising an outer fluid,
   which compartments are separated by a means allowing the exchange of predetermined substances,
(b) providing a cell-free expression system and at least one protein encoding nucleic acid in the inner compartment,
(c) incubating the combined at least one protein encoding nucleic acid and the cell-free expression system under conditions suitable for expressing the at least one protein encoded by the nucleic acid, and
(d) passing of the expressed at least one protein from the inner compartment comprising the inner fluid to the outer compartment comprising the outer fluid,
(e) purification and/or isolation of the expressed at least one protein.

In the context of the invention, continuous expression of at least one protein may be characterized by the fact that at the initiation of expression, not all components are present, or are not present in sufficient quantities, or are not present in the corresponding inner or outer compartment of the reaction space that are required for the provision of the desired end product. Thus, continuous expression may be characterized by the supply of at least one component required for production to at least one of the compartments, wherein this supply step preferably occurs after the initiation of expression.

Supplying to a compartment is to be understood as supplying the respective component either externally or by means of translocation from one compartment to another. The absent component can be supplied based on factors such as time, reaching a specific process step, such as the formation of a particular intermediate product, or achieving a certain concentration of a component involved in the process.

Achieving a specific concentration and/or parameter limit is characterized in this context by reaching or surpassing a defined upper or lower component concentration and/or limit. As herein understood, "concentration" is the abundance of a component divided by the total volume of the mixture or fluid whereas "parameter limit" might define functional parameters such as pH values or ion-strength and the like.

In a preferred embodiment, supplying a component involves replenishing a substance consumed or transformed in the expression process.

In further preferred embodiments, supplying components comprises supplying components which provide energy, supplying enzymes, supplying proteins or protein moieties, supplying co-factors, supplying salts, supplying nucleic acids - including nucleic acids as templates for further use in the continuous expression method as well as nucleic acids like virus genomes for packaging into empty protein hulls of a virus - and supplying compounds for post-translational modification, e.g. protein labelling or glycosylation.

The terms "peptide" and "protein" are understood by the person skilled in the art and may be used interchangeable where appropriate.

The inventive continuous expression method can be used for the production of a variety of proteins and/or protein-moieties. The method is further particularly suited for the production of multi-protein complexes, comprising either identical proteins e.g., homologous multimers, or different proteins or protein moieties e.g., heterologous multimers. Thus, the inventive method comprises also the provision of multi-protein complexes. In general, the term "homologous" as used herein refers to a similar position, structure, amino acid sequence and/or activity, wherein the term "heterologous" refers to a different or clearly distinguishable position, structure, amino acid sequence and/or activity.

For heterologous multimers, the involved proteins can be expressed either in parallel, such as through fusion proteins or by means of bi- or poly-cistronic expression, or sequentially, for example, by template exchange or external supply of at least one participating protein.

The skilled person understands that the method is not only suitable for the expression of the proteins mentioned below, such as cytotoxic proteins, neo-antigens, split-proteins, antibodies or antibody fragments, nanobodies, viral proteins, and bacteriophage proteins. Rather, the person skilled knows how to adapt the method for the production of further proteins without exercising inventive activity. However, the expression, i.e. provision of viruses and in particular bacteriophages (herein interchangeably also called "phages") is preferred.

In a preferred embodiment, the continuous expression method is used for the production of a multi-protein complex being a naturally occurring or synthetic bacteriophage or virus or functional fragment thereof.

Such functional fragment as herein understood may be set up by single monomeric peptides or may be composed of a couple proteins, which may be identical or not, which assemble and/or bind together to form a structure like a capsid, tail or tail fiber. Thus, functional sites may be composed by monomeric peptides, oligomeric peptides as well as several different proteins. For example, capsid structures may be set up of several identical monomeric peptides, T7 tail fiber are set up as a homotrimer of three identical peptides and the T4 long fiber tail is set up of different proteins.

The multi-protein complexes provided by the method of the invention can comprise several different functional sites. Wherein a functional site is preferably selected from head (capsid), tail, spike, sheath, tube, baseplate and tail fiber components of a bacteriophage. Capsid and tail fiber functional sites are in particular preferred. The provided multi-protein complexes can contain a given functional site, i.e. a type of functional site, several times, e.g. one to six times, such as a fiber tail. For example, if the functional site comprising homologous proteins is a bacteriophage head or capsid component, there is one corresponding functional site in the multi-peptide structure; if the functional site comprising homologous proteins is a tail fiber there are usually one to six, preferably six, corresponding functional sites in the multi-peptide structure.

The provided multi-peptide complexes may comprise a given type of functional site at least two times, e.g. like a fiber tail and is preferably selected from the group comprising tail fiber proteins or tail fiber loops and/or capsid proteins, in particular receptor binding proteins. A tail fiber may be composed of homologous proteins including receptor binding proteins. For example, the long tail fiber of *E. coli* phage T4 is composed of four different proteins, wherein the distal subunit of the fiber, gp37, mediates receptor binding with its carboxy-terminal region.

The provided multi-protein complexes may be derived from different bacteriophages, preferably synthetic bacteriophages and may be part of the same functional site for example, tail fiber proteins, tail fiber loops, capsid proteins, receptor binding proteins, etc. in a multi-peptide structure. Tail fiber proteins are in particular preferred.

The provided multi-protein complex preferably resembles a bacteriophage structure; in other words, the multi-protein structure is preferably a "synthetic bacteriophage" or "engineered bacteriophage".

In general, the basic structure of bacteriophages consists of a core of nucleus material, i.e. a nucleic acid, preferably genome, surrounded by a protein capsid (head). Bacteriophages may exist in three basic structural forms, an icosahedral-like head with a tail, an icosahedral-like head without a tail and a filamentous form. Basic structures comprising an icosahedral head with a tail are preferred. However, a multi-protein complex provided herein may also only partly resemble a bacteriophage, i.e. the head may be deleted and/or the remaining bacteria penetration structure may be altered. As understood herein such a "headless bacteriophage" is also comprised by the term synthetic bacteriophage. In particular, such "headless bacteriophages" can induce cell death without replication of the bacteriophage, for example by damaging the membrane potential.

Despite the enormous differences in the shape of viruses, generalized principles for the structure of the protein coat can be found, since it is based on the self-assembly of smaller asymmetric building blocks.

In a preferred embodiment, the continuous expression method is used for the expression of a multi-protein complex comprising at least two homologous tail fiber proteins, preferably derived from different bacteriophages and/or at least two homologous capsid proteins, preferably derived from different bacteriophages.

The multi-protein complex which is a synthetic bacteriophage expressed by the inventive method is preferably self-assembled. "Self-assembled" within the present invention refers to a multi-peptide complex, that has been spontaneously formed to a structural organization or pattern by the specific interaction of involved proteins. Such structural organization may be a synthetic bacteriophage comprising all components of a wild-type bacteriophage, or a synthetic bacteriophage not comprising all components of a wild-type bacteriophage such as a headless bacteriophage. The proteins providing a multi-peptide structure may self-assemble via specific non-covalent interaction, i.e. the proteins providing a self-assembled multi-peptide structure are non-covalently bonded to each other. However, such "self-assembly" might be assisted, for example by chaperons. As herein understood "assembly" and/or "self-assembly" comprises any kind of binding which may be covalent or non-covalent.

The provided multi-protein complex, preferably a synthetic bacteriophage, is preferably capable of binding on the surface of a cell, in particular a bacterial cell. The homologous tail fiber proteins may be specific for the same or different hosts, wherein being specific for different hosts is preferred. The multi-protein complex may be called multi-functional. "Host" as used herein describes the whole breadth of organisms comprising single species as well as strains which can be effected by the multi-protein complex or synthetic bacteriophage expressed by the method of the present invention.

As herein understood tail fiber proteins and in particular homologous tail fiber proteins may be specific for any suitable target. For example, homologous tail fiber proteins may be specific for gram positive and/or gram negative bacteria, in particular gram positive and gram negative bacteria, or even mycobacteria and/or gram positive and/or gram negative bacteria. Having homologous tail fiber proteins and/or receptor binding proteins incorporated, the host range of the inventive multi-peptide complex, in particular synthetic bacteriophage, may be enhanced.

The homologous peptides expressed may be derived from different bacteriophages, in particular different wild-type bacteriophages. However, they may be also derived from the same bacteriophage, wherein at least one peptide has been engineered, or wherein all different homologous peptides are engineered. The expressed bacteriophages may be derived from the same wild-type but engineered in a different way.

Different bacteriophages where the homologous peptides are derived from, in particular wild-type bacteriophages, may be selected from the group comprising Caudovirales (familiy of Myoviridae, Autographiviridae, Podoviridae, Ackermannviridae, and Siphoviridae), Leviviridae, Tectiviridae, Corticoviridae, Plasmaviridae, Sphaerolipoviridae, Inoviridae, Microviridae, Picobirnaviridae and/or Cystoviridae.

According to a preferred embodiment, multi-protein complexes provided herein comprise a bacteriophage derived nucleic acid, in particular bacteriophage genome, encoding at least one homologous peptide less than comprised by the multi-peptide structure expressed by the method of the invention; i.e. after replication, the 1. generation multi-peptide structure, in particular synthetic bacteriophage, differs from the multi-peptide structure as originally expressed by the method of the invention. Thus, the 1. generation multi-peptide structure comprises at least one homologous peptide, being not encoded, less than the originally by the inventive method produced multi-peptide structure.

Such bacteriophage derived nucleic acid may be any genetic material or nuclear material of bacteriophages, in particular bacteriophage a genome, which can be either DNA or RNA and/or variants thereof, which can either be double-stranded or single-stranded. Such genetic material or nuclear material of bacteriophages, in particular of a bacteriophage genome, may also be engineered, or the derived nucleic acid is an unmodified bacteriophage genome, in particular wild-type genome. The genetic material may further facilitate the replication of the multi-protein complex, in particular of the synthetic bacteriophage.

Engineering, e.g. peptide engineering, may expand the abilities of the underlying protein by approaches known to the person skilled in the art and includes in particular site-specific mutations such as insertions, deletions and/or point mutations of the corresponding protein encoding nucleic acids, incorporation of non-natural amino acids as well as the addition of further functional groups such as labeling groups, active agents etc. As understood by the person skilled in the art such engineering can be performed on the nucleic acid level as well as on the protein level.

Due to the degeneracy of the genetic code, any nucleic acid sequence can be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. Such a conservative change may be, for example, done for improved expression of the protein in a given expression system.

According to a preferred embodiment, the provided multi-protein complex comprises receptor binding proteins and/or capsid forming proteins comprising non-naturally generated mutations, e.g. resulting from deletions, insertions and/or substitutions within the corresponding encoding nucleic acid as described above.

According to a preferred embodiment the provide multi-peptide structure, in particular synthetic bacteriophage, may be labelled. Such a label may be, for example, a marker, or an active agent. A "marker" as understood herein may be a tag such as biotin, a his-tag or any other tag recognizable by a binding partner such as an antibody, useable, for example, to isolate the bacteriophage and/or tags masking bacteriophages from the immune system of an object to be treated, such as peptides of human cells to mask the bacteriophage from the human immune system. Markers suitable for use in affinity purification processes include glutathione-5-transferase (GST), protein A, ScFv and lectins. A marker may be also a dye or a fluorescent label, e.g. for use in detection assays. Other modifications of the bacteriophage may be made, e.g. for reducing bacteriophage antigenicity, including use of a PEG (polyethyleneglycol) conjugate or a polysialic acid conjugate. An "active agent" as understood herein enhances the lethality of the bacteriophage to the bacterial host. The label may be incorporated at the DNA level, may be attached chemically at the phage surface or may even self-assemble into the inventive multi-peptide structure, in particular synthetic bacteriophage, within the inventive method described herein.

The continuous expression method of the first aspect of the invention utilizes a liquid-filled partitioned reaction space in which the at least one natural occurring or synthetic protein or multi-protein complex, in particular the virus or bacteriophage, is produced. This reaction space may be a hollow container comprising an inner compartment comprising an inner fluid and an outer compartment comprising an outer fluid, separated by a means enabling the exchange of predetermined substances.

The lateral limits or walls of the compartments of the reaction space are preferably made of a chemically inert material, preferably stainless steel, glass, or plastics such as polypropylene, polyethylene, or polystyrene. They preferably have a volume ranging from a few microliters to several liters, e.g., 1 µl - 100 I.

The inner and outer compartments are separated from each other by means which allow the exchange of predetermined substances. These means are preferably arranged in such a way that they form a common boundary separating the compartments.

Suitable reaction spaces for use in the continuous expression method of the first aspect of the invention can be adapted by those skilled in the art to the specific requirements, and/or selected from a range of commercial systems such as TFF cassettes or Hollow-Fibers such as provided by Sartorius.

The inner compartment comprises the inner fluid which is preferably an aqueous solution, and the outer compartment comprises the outer fluid which is preferably an aqueous solution, and which differs from the inner fluid at least in the concentration of one substance contained therein and/or at least one parameter like pH value, ion strength and/or temperature.

The inner and outer fluids are optionally connected to separate reservoirs in which the corresponding fluid can be stocked so that it can be delivered by means of a fluid stream through the respective inner or outer compartment as required. The reservoirs are preferably made of the same material as the respective compartment and are preferably larger than the latter.

The composition of both fluids may vary during the production process, e.g. by means of dilution, consumption and/or addition of substances, e.g. components as described herein.

In the context of the continuous expression method, a stream, such a supply stream, might be directed through either the inner compartment and/or the outer compartment. The stream or streams can be configured so that, at least temporarily, the inner fluid can be channeled through the outer compartment and/or the outer fluid can be channeled through the inner compartment.

After flowing through the chamber, the liquid is discarded or at least partly reused, preferably by means of a closed circuit which optionally comprises the reservoir.

At the boundary which separates the inner and outer compartments, being preferably a membrane, the inner and outer fluid streams preferably flow in opposite directions, i.e. stream in counter-flow.

The means for separating the compartments are provided by a boundary surface which refers to a thin physical barrier that prevents or at least impedes the passage of specific components from the inner or outer fluid into the respective other compartment.

The boundary surface is preferably made of a porous material that prevents the passage of molecules larger than the pore size. In the context of the invention, this is to be understood as the cut-off value of the boundary surface.

The cut-off value of the boundary surface is preferably chosen to allow the passage of predetermined substances while preventing other substances from translocating into the other compartment due to size exclusion.

Other properties that can contribute to the size-exclusion filtration or alternatively replace it are mainly the shape and structure of the pores, hydrophobic or hydrophilic properties of the boundary, charge of coatings on the boundary. The separation is further influenced by salt content and pH value and ionic strength of the fluids on both sides of the boundary surface as well as by the pressure in the inner or outer compartment. The beneficial utilization and combination of these properties is known to the skilled person from a variety of filtration techniques such as ultrafiltration, dialysis or reverse osmosis and can be adapted to the specific requirements of continuous expression method of the invention without exercising inventive activity.

By adjusting the above-mentioned parameters, the permeability of the membrane can for instance be tailored to allow for the build-up of high concentrations of a specific substance on one side of the membrane.

The boundary surface is preferably a membrane of artificial or biological origin and may incorporate selective transporters or pores for specific substances.

The boundary surface preferably prevents the passage of the expressed protein or multi-protein complex. However, modifying parameters such as pH value, ionic strength, and cut-off value enable the translocation of the expressed protein to the outer compartment, where it can be harvested for further use. This harvested protein or protein-complex is essentially free from at least the nucleic acid template of the inner compartment and preferably also free of other components of the reaction mix.

It is preferred that the at least one protein expressed and/or assembled in the inner compartment translocates to the outer compartment where it is collected and/or optionally (i) interacts with, (ii) is modified by, or (iii) encapsulates substances provided in the outer fluid of the outer compartment.

The compartments can be optionally configured in such a way that the inlet and/or outlet of the inner and/or outer chamber or reservoir is equipped with a filter which prevents predetermined substances from entering or leaving. "Predetermined substances" as herein understood are substances which can be exchanged via the membrane. Before the process according to the invention is carried out, it is determined which substances are to pass through the membrane during the process, in other words, should be able to change from the inner compartments to the outer compartment and be converted. Based on these predetermined substances, the membrane used or its characteristics, respectively, such as cut-off value, hydrophilicity or hydrophobic characteristics etc. may be selected.

The skilled person can determine these substances on the basis of the selected coding nucleic acids, the selected reaction mix and the desired product before starting the expression and plan their addition or removal accordingly.

Due to a lack of cell walls and cell membranes, the open configuration of the reaction space of the continuous CFPS method of the invention may provide direct access to the production environment allowing for easy modulation of basic parameters such as pH, temperature and concentrations of ions and cofactors.

In addition, components as herein described as well as non-natural components such as non-natural or chemically modified amino acids, specific reaction mixes containing non-natural or modified substrates or components for post-translational modification of the proteins produced can easily be introduced into the open reaction space if required.

An open configuration of the reaction space of the inventive continuous CFPS method system also allows for the selective retrieval of samples for monitoring and optimization of the cell-free protein production process. Samples can be obtained and analyzed at any time, which allows to measure the kinetics of the production process in relation to fluctuating parameters such as the availability of specific components and thus to adapt and/or improve the production process in a targeted manner. This is particularly useful for fine-tuning conditions for challenging proteins or when multiple proteins are expressed in parallel, e.g. in the cell-free synthesis of viruses or bacteriophages.

The ability to easily manipulate the chemical environment makes continuous CFPS method suitable for high-throughput screening applications, as it allows to rapidly screen different reaction conditions and components to identify optimal conditions for protein expression as well as for functional genomics studies where combinations of genes are expressed, and respective protein combinations are studied in a controlled environment.

The terms "cell-free expression system" or "cell-free protein synthesis system" (CFPS system) are well understood by a person skilled in the art and refers in general to an in-vitro method that allows the synthesis of proteins outside of living cells.

CFPS systems are not only suitable for the production of individual proteins or protein moieties, but also for the parallel production of multiple distinct proteins that are either independent of each other or assemble into higher multiprotein units such as bacteriophage hulls. In the context of the present invention, the CFPS system is a preferably host independent system. The term "cell free" is understood by the person skilled in the art and refers to "substantially free of'.

A CFPS system as understood herein comprises a complete transcription and translation machinery for transcription and expression of a virus or bacteriophage nucleic acid, in particular a bacteriophage genome which may, of course, be also modified.

A preferred example for a CFPS system is a cell lysate. Using such a cell lysate it is possible to synthesize several proteins or metabolites at the same time.

"Cell lysate" according to the present invention refers to a fluid comprising the components of cells from which it is derived after lysis. Lysis methods are known to the person skilled in the art and break down the membrane of a cell, for example, by viral, enzymatic, or osmotic mechanisms that compromise its integrity. Such cell lysate is essentially void of intact cells, i.e. cell-free. The terms "cell lysate" and "cell extract" can be used herein interchangeably. After purification this lysate is essentially free, preferably free, of host DNA and makes an expression of the desired protein possible by the external addition of DNA, in particular a amplified bacteriophage genome as herein described. Thus, the used cell lysate is preferably free of nucleic acids, in particular DNA, and/or membranes from the cells of which it is derived. In a preferred embodiment, using standard techniques no host DNA can be detected in the purified lysate.

Cell lysates used according to the present invention may be derived from microorganisms in particular bacteria such non-pathogenic or pathogenic bacteria (for example *E. coli*), imperfect or perfect fungi such as yeast, or from eukaryotic cells such as insect, mammalian and/or plant cells (in particular wheat or rice), or may be even artificially. produced. "Microorganism" refers to a bacterium or an archaeon. Preferably, the microorganism is a bacterium.

A number of cell-free expression systems are known to a person skilled in the art und may be applied according to the present invention. For example, the "PURE" system consists of several isolated proteins (Shimizu et al.) while untreated cell lysates (crude extracts) such as of *E*. *coli* include almost all intracellular proteins, even those that are not necessary for expression.

The use of *E. coli* lysate, in particular crude *E. coli* lysate, is a further preferred embodiment. A preferred example of crude *E. coli* lysate is *E*. *coli* S30 cell extract produced by a method based on the protocol described in E. Falgenhauer et al. (Falgenhauer, S. von Schönberg, C. Meng, A. Möckl, K. Vogele, Q. Emslander, C. Ludwig, F. C. Simmel, ChemBioChem 2021, 22, 2805).

External factors such as energy carriers (e.g. 3-phosphoglyceric acid), co-factors, natural and/or non-natural amino acids, polymerases, transcription regulatory factors, chaperons, DNA stabilization agents (e.g. GamS and/or Chi6 DNA) may be added to enhance or otherwise improve the reaction. Such factors may be added as proteins and/or as nucleic acid encoding a respective protein.

According to a preferred embodiment, polyethylene glycol (PEG, in particular PEG 8000) and/or Ficoll (e.g.Ficoll-400) may be added to the cell extract to increase molecular crowding. PEG binds water, creating a more concentrated environment. This may be advantageous because there is up to 25 to 30 times less protein concentration in the cell extract than in the cytosol of a bacterial cell.

If the cell-free lysate to be used is derived from a cell or microorganism which is different to the natural host of the virus or bacteriophage, at least one bacteriophage-host specific expression factor and/or a nucleic acid encoding the at least one bacteriophage-host specific expression factor, preferably a transcription factor, may be added, if necessary. "Bacteriophage specific host factor" or "bacteriophage-host specific expression factor" with regard to a given bacteriophage are well known to the person skilled in the art and/or can be easily determined (cf e.g. Role of host factors in bacteriophage φ29 DNA replication, Adv Virus Res 2012; 82:351-83 or US2022025957A1).

According to one embodiment of the invention, at least one nucleotide sequence encoding the at least one bacteriophage-host specific expression factor or any other protein preferably selected from the group comprising co-factors, chaperons, polymerases, transcription regulatory factors is cloned into the cell strain used to provide the cell lysate. The nucleotide sequence is expressed within the cell before lysis. Thus, after lysis the cell lysate already contains all nucleic acids and/or proteins necessary for the amplification of the virus or bacteriophage, in particular at least one host specific expression factor or any other protein selected from the group comprising co-factors, chaperons, polymerases, transcription regulatory factors and/or any mixture thereof.

In the context of the continuous CFPS method of the invention, the provision of a CFPS system is therefore to be understood in such a way that the reaction chamber, preferably the inner compartment, comprises the CFPS system or parts of the CFPS system. Missing or consumed or depleted substances can be added at any time, usually depending on the state of the reaction, preferably by means of a supply stream.

Further preferred modifications of the CFPS system include the addition of one or more modified proteins or the addition of a nucleic acid encoding these proteins. These proteins may have a natural amino acid sequence or may be artificially modified for optimization and may be incorporated into the multiprotein complex, which is preferably a bacteriophage, or may modify it, e.g. by an enzymatic modification such as glycosylation.

In the context of the continuous CFPS method of the invention, the protein synthesis is maintained over a prolonged period of time in order, for instance to increase the yield of the end product, such as the desired bacteriophages.

To enable maintaining the synthesis inside the compartment throughout the incubation period, protein expression needs replenishment of consumed substances such as nucleotides or energy carriers. The skilled person can deduce which compounds are required at a given time by monitoring the reaction or from known production kinetics. These substances therefore represent a part of the predetermined substances that are exchanged via the boundary surface of the reaction chamber, especially if the replenishment is not realized by means of a supply stream running through the inner compartment, but by a supply stream running past the outer boundary surface of the compartment.

Which substances can be exchanged from one compartment to another using the continuous expression method of the invention also depends on the specific material properties of the boundary surface. The skilled person will therefore tailor the boundary material to suit the method. In a broad sense, predetermined substances in the context of the invention therefore refers to all substances that have to be exchanged across the boundary surface during protein production in order to produce the desired protein but also the expressed proteins itself.

The term "predetermined substances" therefore preferably comprise the provided proteins itself but also external factors such as energy carriers, co-factors (likewise PEG e.g. to increase molecular crowding), amino acids (natural occurring and/or synthetic amino acids comprising nuclease resistant and labeled amino acids), polymerases, ligases, transcription regulatory factors, chaperons, DNA stabilization agents (e.g. GamS and/or Chi6 DNA). Such factors may be added as proteins and/or as nucleic acid encoding a respective protein.

Preferred are also substances and components enabling post-translational protein modification such as glycosylation, acylation, alkylation, carboxylation, hydroxylation, methylation, myristoylation, palmitoylation, isoprenylation, lipoylation or substances for the specific labelling of proteins such as chemical dyes or protein tags (e.g. affinity or snap tags) to be exchanged via the membrane as required.

Furthermore, proteins produced in the reaction chamber are preferably exchanged across the boundary and are preferably isolated with high purity from the outer fluid or interact with substances provided therein. Preferably, specific post-translational modifications and/or labelling are added to the provided protein in the outer fluid.

The term "protein encoding nucleic acid" is well understood by the persons skilled in the art and refers to a segment of nucleic acid which can either be DNA or RNA that contains the genetic information necessary for the synthesis of a protein. In the context of molecular biology and genetics. In essence, the protein-encoding nucleic acid carries the genetic code or sequence that is translated into a corresponding protein. In eukaryotic organisms, such as humans, this process involves transcription of DNA into mRNA (messenger RNA) and then translation of mRNA into a specific sequence of amino acids, forming a protein. In a basic form, a nucleic acid encoding a protein comprises a promotor, a start codon, the open reading frame of the coding region, and a stop codon.

However, the person skilled in the art is well aware that a nucleic acid encoding an amino acid can be provided by various means, comprising circular or linear forms of either single or double stranded DNA or RNA, including PCR products, plasmids or vectors, e.g. based on modified chromosomes or by means of providing whole naturally or artificial chromosomes or genomes. For the production of inventive multi-protein complexes, a nucleic acid can for instance be provided which codes for a fusion protein on the basis of already fused "open reading frames".

However, the proteins involved in the formation of a multi-protein complex can also be expressed in parallel or sequentially, both mono- and/or poly-cistronic, and can assemble into multi-protein complexes independently or by means of additionally provided co-factors such as chaperones.

Many suitable methods for the provision and modification of coding nucleic acids are available in the art. One of the more recent developments comprises the CRISPR/Cas9 technology. A revolutionary gene-editing technology that allows for precise modification of DNA. The CRISPR system utilizes RNA molecules to target specific DNA sequences, and the enzyme Cas9 cuts the DNA at those locations. This cut triggers the cell's repair mechanisms, enabling the introduction of genetic changes. CRISPR/Cas9 is employed in genome editing, genetic research, and the development of novel therapies.

By way of in the example, for the provision of encoding nucleic acids, reference is made to one of many widely recognized publications "Molecular Cloning: A Laboratory Manual" by Michael J. Sambrook and David W Russell.

In addition to the genome of a wild-type bacteriophage, a genome modified by any of the methods known in the art can also be used for the expression of viruses or bacteriophages in the continuous expression method of the invention.

Preferably, the DNA and/or RNA molecules for use in the method of the invention are essentially complete genomes of different bacteriophages or protein encoding regions thereof, preferably phage tail fiber proteins and/or homologous phage capsid proteins.

It is further envisaged that the nucleic acid of at least two different wild-type bacteriophages, a first wild-type bacteriophage and at least one mutated or engineered bacteriophage derived from said first wild-type bacteriophage or at least two mutated or engineered bacteriophages derived from the same wild-type bacteriophage is provides for use in the method of the invention. Varying the concentration of the thereby added coding genetic information, it is possible to adjust the final concentration of the resulting multi-peptide structure for further use.

When providing protein encoding nucleic acids of at least two different bacteriophages the encoding nucleic acid may be modified, in particular modified by non-naturally mutations such as deletion, insertion and/ or substitution as described herein above.

When providing protein encoding nucleic acids of at least two different bacteriophages it is preferred that at least one of the encoded proteins is preferably a capsid protein and/or tail fiber protein.

The concept of incubating the combined at least one protein encoding nucleic acid and the cell-free expression system under conditions suitable for expressing the at least one protein encoded by the nucleic acid is generally understood by a person skilled in the art. For stability reasons, the nucleic acid and the cell-free expression system are usually stored at low temperatures until use. The incubation is preferably started by mixing the coding nucleic acid with the cell-free reaction mix.

Such mixing is preferably carried out in the inner compartment of the reaction space. Temperature and parameters such as pH-Value or salt concentrations of the fluid are preferably chosen to resemble physiological values for the organism or the cells from which the cell-lysate was obtained, and wherein the incubation, and preferably also the continuous expression of the at least one protein can persist for an extended period, e.g. from a few minutes to several days; preferably from 5 minutes to 100 hours, preferably 10h to 80h, more preferably 24h to 72h, most preferably 40h to 60h and in particular about 48h.

The process according to the invention is generally carried out below 37°C; usually in the range of 20°C to 30°C. In other embodiments, however, lower temperatures such as 4°C are also possible.

The protein, multi-protein complex, virus or bacteriophage expressed in the continuous CFPS method of the invention is provided preferably in an isolated form. Corresponding techniques for isolating or purifying are known to the person skilled in the art. The provided protein can be purified and/or isolated from the inner fluid and/or the outer fluid, wherein the outer fluid is preferred.

The concentration of the proteins, multi-peptide structures, in particular bacteriophages, generated using the continuous CFPS method may be at a level that the corresponding composition and/or formulation can directly be applied in a therapy with phages without a further concentration step. If needed, a further concentration step, in particular after purification, is of course also within the scope of the present invention.

A further aspect of the invention relates to a multi-peptide structure, in particular a synthetic bacteriophage, provided by the continuous CFPS method methods described herein. All embodiments described herein relate preferably to the production of self-assembled multi-peptide structures, in particular synthetic bacteriophages.

Another aspect of the invention relates to a composition comprising the multi-peptide structure, in particular synthetic bacteriophage, provided by the continuous CFPS method as described herein.

A further aspect of the invention relates to a composition comprising two or more types of multi-peptide structures, in particular synthetic bacteriophages, provided by the continuous CFPS method as described herein, wherein the two or more types of the multi-peptide structure, in particular synthetic bacteriophage, have homologous tail fiber and/or capsid proteins or have different mutations in the tail fiber and/or capsid proteins. Such a composition comprises different synthetic bacteriophages which may have a statistical combination of the functional sites set up by the at least two homologous peptide, such as a statistical combination of tail fiber with regard to their protein composition as well as their positioning in the multi-peptide structure. It is preferred that the two or more types of the multi-peptide structure, in particular synthetic bacteriophage, have different host ranges.

The compositions comprising bacteriophages provided by the method of the invention may comprise carriers and/or vehicles as described below. According to preferred embodiments, the compositions are pharmaceutical compositions.

A further aspect of the invention relates to the multi-peptide structure, in particular synthetic bacteriophage, provided by the continuous CFPS method as described herein for use in medicine, i.e. as a medicament, chemistry, biotechnology, agriculture and/or food industry. Such uses are not limited to bacteria related fields and comprise, for example, the use as vaccine, for example against infections as well as tumors, and the use as a delivery vehicle for any kind of drug, in particular anti-cancer drugs, and/or nucleic acids to a target cell, in particular a human target cell. A preferred embodiment relates to the use of a multi-peptide structure, in particular synthetic bacteriophage, provided as described herein for use as a medicament. According to certain embodiments the multi-peptide structure, in particular synthetic bacteriophage, described herein may serve as vehicle or carrier for antigens to be delivered. For example, such antigen structures to be delivered may be attached to the capsid structure of a synthetic bacteriophage. The multi-peptide structure, in particular synthetic bacteriophage, may serve as or provide vaccines, e.g. by delivery of antigens and/or carrying nucleic acids to be expressed.

According to preferred embodiments, the multi-peptide structures, in particular synthetic bacteriophages, provided by the continuous CFPS method as well as compositions described herein are used to infect targets, in particular hosts, in a first infection cycle. Thereby it may be avoided that the multi-peptide structures, in particular synthetic bacteriophages, revert to their original non-engineered state after one round of infection.

The use in bacteria related fields is, however, preferred. Multi-peptide structures, in particular synthetic bacteriophages provided by the continuous CFPS method, may in particular be used as bacterial population control alternatives to antibiotics. They are much more specific than antibiotics and are typically harmless not only to the host organism but also to other beneficial bacteria, such as the gut microbiota, as they are very selective in the strains of bacteria, they are effective against. Thereby the chances of opportunistic infections are significantly reduced. Advantages include further reduced side-effects and reduced risk of the bacteria developing resistance. Because phages replicate in vivo, in certain embodiments small effective doses can be used. These unique properties make them highly promising antimicrobials.

Accordingly, the multi-peptide structures, in particular synthetic bacteriophages, provided by the continuous CFPS method described herein may be part of a pharmaceutical composition. Such a composition may comprise (i) at least one bacteriophage strain capable of producing a lytic infection and (ii) a pharmaceutically acceptable carrier and/or vehicle. An inventive composition may also comprise (i) at least one (synthetic) bacteriophage structure protein and (ii) a pharmaceutically acceptable carrier and/or vehicle and/or therapeutic agent. Of course, such compositions may comprise synthetic bacteriophage mixtures. Preferred embodiments relate to such compositions for use as a medicament.

The pharmaceutical compositions may be used in combination with antibiotics for the purpose of treating bacterial infections and/or to treat antibiotic resistant bacteria. In particular, bacteriophages tend to be more successful than antibiotics where there is a biofilm covered by a polysaccharide layer, which antibiotics typically cannot penetrate. According to a preferred embodiment, the composition may be freeze dried.

"Pharmaceutically acceptable carrier" relates to pharmaceutically-acceptable, fillers or diluents used to formulate pharmaceutical compositions for animal or human administration. The pharmaceutical compositions may further comprise pharmaceutically acceptable auxiliary agents, and optionally other therapeutic agents. In particular for oral administration it is preferred to add an antacid, i.e. a substance which neutralizes stomach acidity, thereby increasing the number of phages surviving passage through the stomach.

"Vehicle" as used herein refers to any compound or combination known to the person skilled in the art known to be useful in formulating a composition, in particular a pharmaceutical composition. Such a vehicle may include one or more substances which may also act as flavoring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material.

In tablets, the active agent (i.e. multi-peptide structure, in particular bacteriophage) may be mixed with a vehicle having the necessary compression properties in suitable proportions and compacted in the shape and size desired.

Suitable solid vehicles include, for example calcium phosphate, magnesium stearate, talc, sugars, lactose, dextrin, starch, gelatin, cellulose, polyvinylpyrrolidone, low melting waxes and ion exchange resins.

In another embodiment, the pharmaceutical vehicle may be a gel and the composition may be in the form of a cream or the like.

The dose and regimen of administration of a pharmaceutical composition will necessarily be dependent upon the therapeutic effect to be achieved (e.g. treatment of IBD) and may vary with the particular bacteriophage strains in the composition, the route of administration, and the age and condition of the individual subject to whom the pharmaceutical composition is to be administered.

If the pharmaceutical composition is to be administered with one or more antibiotics, it may be simultaneously, separately or sequentially administered.

Pharmaceutical compositions and routes of administration include those suitable for or via oral (including buccal, sublingual and intraorbital), rectal, nasal, topical (including transdermal), ocular, vaginal, bronchial, pulmonary or parenteral (including subcutaneous, intramuscular, intravenous, intradermal, intraperitoneal, intrapleural, intravesicular and intrathecal) administration or administration via an implant. In particular a freeze-dried composition may be administered orally, preferably in form of a pill.

Agriculture comprises plant agriculture as well as animal agriculture, wherein bacteriophages can replace or complement antibiotics and/or pesticides.

Bacterial pathogens are associated with a couple of plant diseases which can also be treated with synthetic bacteriophages provided herein. Non limiting examples for bacterial plant diseases which might be treated are leaf blight on onion, potato scab or soft rot of potato, bacterial wilt on tobacco, citrus bacterial spot or citrus canker of citrus, fire blight on apple, black rot of cabbage, soft rot of calla lilies, bacterial wilt of tomato and bacterial spot of peach.

The use of the multi-peptide structures, in particular synthetic bacteriophages, provided by the continuous CFPS method in animal agriculture is exemplified by the use against Salmonella, pathogenic E. coli, Clostridium, and Campylobacter for the poultry industry and against Salmonella and pathogenic E. coli for the pig industry.

Multi-peptide structures, in particular synthetic bacteriophages, provided by the continuous CFPS method may also be used to safen food products, and to forestall spoilage bacteria. Since 2006, the United States Food and Drug Administration (FDA) and United States Department of Agriculture (USDA) have approved several bacteriophage products. For example, LMP-102 (Intralytix) was approved for treating ready-to-eat (RTE) poultry and meat products. In that same year, the FDA approved LISTEX using bacteriophages on cheese to kill Listeria monocytogenes bacteria.

With regard to biotechnology related uses, the methods described herein as well as multi-peptide structures, in particular synthetic bacteriophages, provided herein can, for example, be used for phage display related application.

The invention further includes a kit comprising a pharmaceutical composition of the invention and instructions for the use of the composition for a use as hereinbefore described, optionally together with packaging material.

Further aspects relate to the use of multi-peptide structures, in particular synthetic bacteriophages, provided by methods of the invention for the prevention and or treatment of a bacterial infection as well as for avoiding bacterial growth.

### Figures

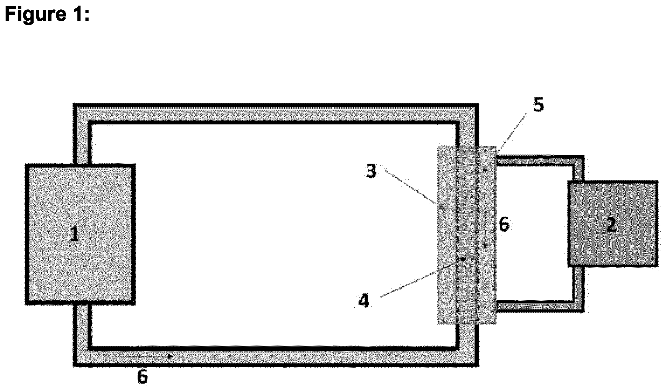

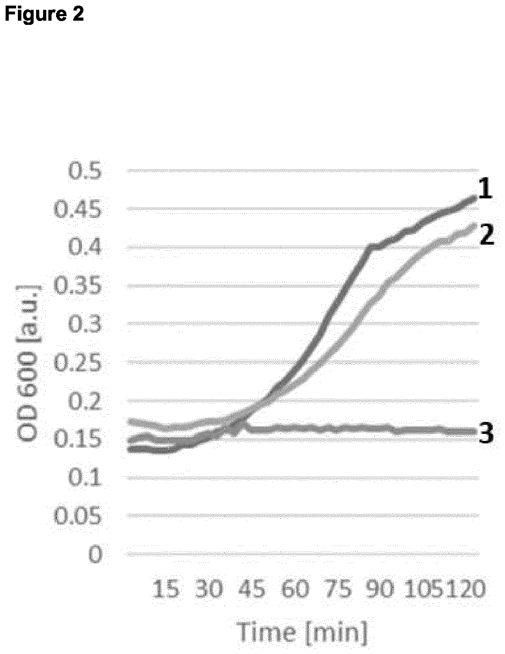

- **Figure 1:**: **Schematic representation of a TFF system used according to the invention and used in Example 1**
1) Reservoir of the inner compartment
2) Reservoir of the outer compartment
3) Outer fluid/solution comprising for example RNA encoding for GFP and nutrients (`buffer b')
4) Inner fluid/solution comprising cell-free reaction mix and protein encoding nucleic acid, such as MS2 phage genome
5) means for separating compartments, such as TFF hollow-fiber with a cut-off of 30 KDA
6) Arrow indicates flow direction
- **Figure 2:**: **Growth curve of host bacteria**
Subsequent to the expression of phage capsid proteins in a TFF reaction space a sample of the outer fluid (1) and a sample of the inner fluid (2) were mixed with host bacteria adjusted to a an OD600 of 0.15 and bacterial growth (OD600) was followed for 120 min. Increasing OD600 values in (1) and (2) indicate comparable growth rates in both samples. (3) control; reaction mix without host bacteria.

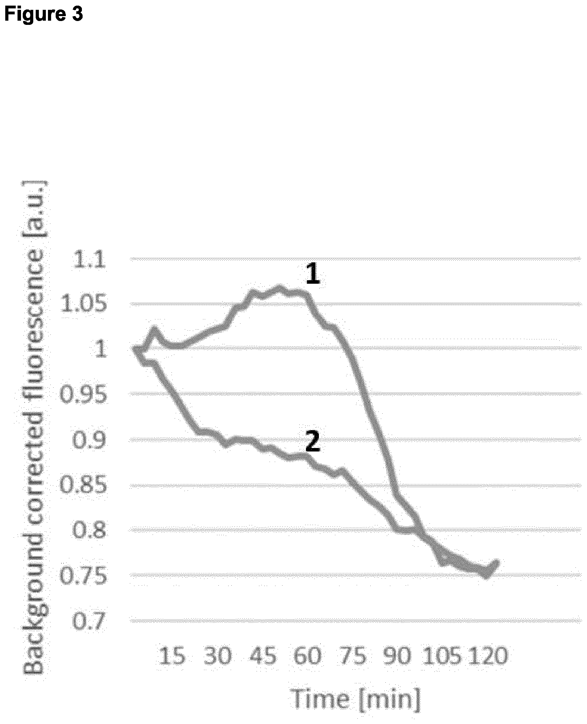

- **Figure 3:**: **External RNA template dependent fluorescence**
Subsequent to the expression of phage capsid proteins in a TFF reaction space a sample of the outer fluid (1) and a sample of the inner fluid (2) were mixed with host bacteria and the respective fluorescence of samples was followed for 120 min. Signal intensity was normalized using a sample comprising only the reaction mix but no encoding genetic material.
Fluorescence values alter over time, dependent on expression of GFP in sample (1) and growth dependent consumption of fluorescence emitting NADH in sample (1) and (2). Initial increase indicates that GFP is only expressed in the sample from the outer compartment.

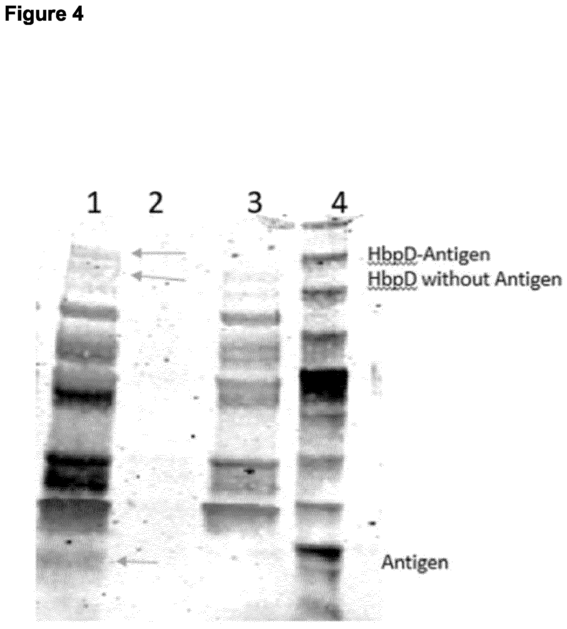

**Figure 4: Western blot of decorated and non-decorated OMV proteins**
Denaturing western blot following the cell-free expression of a SpyCatcher-fused antigen in the inner chamber of a TFF reaction space, with the simultaneous presence of a Salmonella OMV decorated with an Hbp-Spy-Tag in the outer chamber. Outer and inner reaction chambers were separated by a membrane with a 30 kDa cut-off. **(1)** outer fluid comprising OMV after 12 hours of incubation; **(2)** outer fluid without OMV (negative control); **(3)** outer fluid with OMV at start of expression; **(4)** broad range protein-size standard (NEB)
The arrow in the low molecular weight range indicates uncoupled antigen protein. Arrows in the high molecular weight range indicate uncoupled Hbp protein and covalently bound (heavier) antigen-Hbp protein complex.

The present invention is in particular described by the following items:
1. Method for continuous expression of at least one protein in a cell free expression system comprising:
   (a) providing a fluid-filled reaction space
      comprising an inner compartment comprising an inner fluid and
      an outer compartment comprising an outer fluid,
      which compartments are separated by a means allowing the exchange of predetermined substances,
   (b) providing a cell-free expression system and at least one protein encoding nucleic acid in the inner compartment,
   (c) incubating the combined at least one protein encoding nucleic acid and the cell-free expression system under conditions suitable for expressing the at least one protein encoded by the nucleic acid, and
   (d) passing of the expressed at least one protein from the inner compartment comprising the inner fluid to the outer compartment comprising the outer fluid, and optional
   (e) purification and/or isolation of the expressed at least one protein.
2. The method according to item 1,
   wherein the nucleic acid provided in step (b) encodes one or more naturally occurring or synthetic proteins selected from the group comprising: cytotoxic proteins, neo-antigens, split-proteins, nanobodies, viral proteins, and bacteriophage proteins, and/or wherein the nucleic acid of (b) is a naturally occurring or altered virus or bacteriophage encoding nucleic acid, in particular genome.
3. The method according to item 1 or 2,
   wherein assembling, binding and/or self-assembling of the expressed proteins to multi-protein structures takes place in the inner compartment and wherein the expressed proteins remain in the inner fluid until purification and/or isolation.
4. The method according to any of items 1-2,
   wherein assembling, binding and/or self-assembling of the expressed proteins to multi-protein structures takes place in the outer compartment.
5. The method of any of items 1 - 4,
   wherein the inner and the outer compartment are at least partially separated by a membrane.
6. The method of any of items 1-5,
   wherein the inner compartment comprises a membrane which differs, preferably by its cut-off value, from the means, being preferably a membrane, separating the inner and outer compartment.
7. The method of any of items 1-6,
   wherein the reaction space comprises at least one hollow fiber, or TFF Flat sheet cassettes.
8. The method of any of items 1 - 7,
   wherein an exchange of predetermined substances occurs across the means, preferably membrane, separating the outer compartment and the inner compartment.
9. The method of any of items 1 - 8,
   wherein the exchange of substances is provided by means of dialysis or tangential flow filtration.
10. The method of any of items 1 - 9,
   wherein the outer fluid and the inner fluid are based on the same solution or different solutions.
11. The method of any of items 1 - 10,
   wherein the outer fluid is directed to flow around or along the inner compartment or is directed to flow through the inner compartment.
12. The method of any of items 1 - 11,
   wherein the inner fluid and the outer fluid are guided in counterflow, preferably in continuous flow-streams, or non-continuous flow-streams, e.g. sequentially.
13. The method of any of items 1 - 12,
   comprising a step of adding of at least one additive supplementing, supporting and/or modulating protein expression, improving protein stability and/or protein assembly or self-assembly, to the inner compartment and/or to the outer compartment.
14. The method of any of items 1-13,
   comprising a step of adding of at least one additive supplementing, supporting and/or modulating protein expression to the inner compartment, preferably selected from natural or non-natural amino acids, co-factors, tRNAs, GTP, chaperons, polymerases, transcription regulatory factors, and/or any mixtures thereof.
15. The method of any of items 1-14,
   comprising a step of adding of at least one additive enhancing protein assembly or self-assembly to the outer compartment, e.g. chaperons, and/or posttranslational protein modification, such as enzymes and substrates for acylation, alkylation, carboxylation, hydroxylation, methylation, myristoylation, palmitoylation, isoprenylation, lipoylation, glycosylation, etc.
16. The method of any of items 13 - 15,
   wherein the additives are added continuously or non-continuously.
17. The method of any of items 1-16,
   wherein the outer fluid is continuously supplied to the inner compartment, e.g. via a continuous supply-stream, or sequentially, e.g. being part of a multi-stage process.
18. The method of any of items 1-17,
   wherein nucleic acids and/or additives removed from the inner compartment are at least partially reused or recycled.
19. The method of any of items 1-18,
   which is a replenishing process.
20. The method of any of items 1 - 19,
   wherein the cell-free expression system is host independent and preferably selected from cell lysates or artificial expression systems.
21. The method of any of items 1 - 20,
   wherein the cell lysates are derived from microorganisms, in particular *E. coli,* yeast, insects, mammals, plants and/or are artificial.
22. The method any of items 1-21,
   wherein a modified bacteriophage is provided at step (b) by applying at least one step selected of the group:
   (i) providing a modified bacteriophage nucleic acid, in particular genome,
   (ii) adding a modified or unmodified protein to be assembled into the bacteriophage to be provided that differs from the corresponding protein in the corresponding unmodified bacteriophage,
   (iv) adding a nucleic acid encoding for a modified or unmodified protein to be assembled into the bacteriophage to be provided, wherein the protein differs from the corresponding protein in the corresponding unmodified bacteriophage,
   (v) a nucleic acid molecule that suppresses the expression of a protein of a bacteriophage, and/or
   (vi) any combination of steps (i)-(v) thereof.
23. System adapted for the method according to any of items 1-22,
   comprising a reaction space which comprises an inner compartment for taking up an inner fluid and an outer compartment for taking up an outer fluid, which compartments are separated by a means allowing the exchange of predetermined substances.
24. Protein, in particular virus, or bacteriophage, provided by the method of any of items 1 to 22.
25. An all *in-vitro* method according to any of items 1 - 22 for the amplification and provision of synthetic bacteriophages.
26. Protein, in particular virus, or bacteriophage according to item 24 or provided by the method of any of items 1 - 22 or 24 for use in medicine in particular in a method of treatment, prevention and/or vaccination of a patient in need thereof, as well as in, chemistry, biotechnology, agriculture and/or food industry.
27. Protein of item 24 for use in the preparation of a decorated outer membrane vesicle (OMV) serving as an antigen in the formulation of a vaccine.

### Example 1: Preparation of a protein capsid encapsulating foreign RNA

The MS2 phage is a positive-sense single-stranded RNA virus that infects E. coli. The 3.569 nucleotide long genome encodes four proteins of which the maturation protein and the coat protein are of particular interest as they are capable of assembling into an icosahedral shell which encapsulates the RNA genome.

MS2 phage proteins were expressed in a TFF reaction chamber (Fig. 1) comprising a holo-fiber with a cut-off of 30 kDA for separating the outer and the inner compartment. The 30 kDA cut-off value ensures permeability for proteins expressed in the inner compartment while retaining the phage RNA in the inner compartment. The inner solution comprised a TXTL system (cell-free reaction mix) and the MS2 phage genome. The outer compartment comprised RNA encoding for the green fluorescent protein (GFP) and `buffer b' (nutrients).

To evaluate if the GFP encoding RNA, located in the outer solution of the TFF chamber, was packed into the MS2 phage capsid expressed in the inner solution of the TFF reaction chamber, fluorescence was measured in the presence host-bacteria adjusted to an OD600 of approx. 0.15 and mixed with the outer solution of the TFF chamber. As a reference, the inner solution of the TFF chamber comprising MS2 phage particles expressed in the TXTL system encapsulating the native MS2 RNA genome was mixed with host-bacteria.

An increase in OD600 (Fig. 2) in both the outer and inner samples indicates bacterial growth, while the OD600 of a control sample containing no bacteria remains at base level.

The temporal course of the fluorescence measurement (Fig. 3) of the inner and outer sample can be explained as follows. During the uniform growth of the bacteria in both samples, energy is consumed and thus auto-fluorescent NADH is converted into non-fluorescent NAD.

This loss of fluorescence is initially overcompensated in the outer sample, which contains RNA coding for GFP.

The subsequent seen decrease in fluorescence, also in the sample from the outer compartment, reflects the successful encapsulation of the coding RNA into newly formed phage particles.

A functional plaque assay proved in addition that functional phages (indicated by 5 plaques) were only formed in the sample from the inner liquid, while no plaques were formed in the sample from the outer liquid.

The experiment confirms that the phage capsid monomers expressed inside the TFF reaction space translocate to the outside, where they encapsulate the therein provided coding nucleic acid.

Following this method, a population of a pure single phage specimen can be created independent of the expression inside of the reaction chamber. Without the dividing membrane (where RNA cannot pass) a mixture of particles would be created, containing either the RNA sequence of interest or the RNA sequence of the expressed capsid structure.

### Example 2: Preparation of an outer membrane vesicle decorated with antigens

Outer membrane vesicles (OMVs) are small (usually <250 nm) spherical particles, spontaneously released by many Gram-negative bacteria during growth. Naturally occurring OMVs can be used as vaccines or vaccine adjuvants because they elicit a strong immune response and present multiple antigens in their native conformation (L. van der Pol et al.; Biotechnol J. 2015 Sep;10(11):1689-706).

Recently a semisynthetic OMV platform utilizing the Escherichia coli autotransporter *Hbp* (hemoglobin protease) as membrane anchor for the presentation of surface bound antigens was introduced (HB van den Berg van Saparoea et al.; Appl Environ Microbiol. 2018) This system allows any antigen (provided it has been fused with an affinity tag) to be captured by the corresponding Hbp-fused affinity tag counterpart (e.g. of the SpyTag/SpyCatcher system) and to be presented on the surface of the OMV.

Since "spy-tag" fused antigens are expressed separately from the OMV spheres and only bound later to their respective membrane anchored "catcher", these antigens, especially if prone to aggregation, are often not available in native form for binding and subsequent surface presentation.

To avoid aggregation, we expressed a Spy-tag-fused neo-antigen in a TFF reaction chamber. (schematic setup according to Fig. 1) with an inner compartment comprising the nucleic acid encoding the fused neoantigen and the reaction mix, and in the outer compartment comprising mainly the catcher fused Hbp OMV sphere and a hollow fiber with a cut-off of 30 kDa separating the outer and inner compartment. The 30 kDa cut-off value ensures permeability for the Spy-neo-antigen fusion protein expressed in the inner compartment while retaining the OMV with membrane-anchored Spy-catcher fused Hbp protein in the outer compartment.

Subsequently, the antigen distribution in outer compartments of the TFF expression chamber was compared at the start and after the completion of expression using a Western blot (detection based on the HA-tag, which occurs in both the antigen and the Hbp membrane anchor). The size shift of the HbpD-antigen signal towards a higher molecular weight indicates the successful binding of the antigen to the membrane anchored protein in the outer compartment of the TFF expression chamber.

The TFF expression system with inner and outer compartments is therefore suitable for the expression and immediate binding of antigens that, for example, are difficult to express due to a high tendency for aggregation. Accordingly, antigen decorated OMVs produced in the outer compartment can be used for the production of vaccines.

## Claims

1. Method for continuous expression of at least one protein in a cell free expression system comprising:
(f) providing a fluid-filled reaction space
comprising an inner compartment comprising an inner fluid and
an outer compartment comprising an outer fluid,
which compartments are separated by a means allowing the exchange of predetermined substances,
(g) providing a cell-free expression system and at least one protein encoding nucleic acid in the inner compartment,
(h) incubating the combined at least one protein encoding nucleic acid and the cell-free expression system under conditions suitable for expressing the at least one protein encoded by the nucleic acid, and
(i) passing of the expressed at least one protein from the inner compartment comprising the inner fluid to the outer compartment comprising the outer fluid, and optionally
(j) purification and/or isolation of the expressed at least one protein.

2. The method according to claim 1,
wherein the nucleic acid provided in step (b) encodes one or more naturally occurring or synthetic proteins selected from the group comprising: cytotoxic proteins, neo-antigens, split-proteins, nanobodies, viral proteins, and bacteriophage proteins, and/or wherein the nucleic acid of (b) is a naturally occurring or altered virus or bacteriophage nucleic acid, in particular genome.

3. The method according to claim 1 or 2,
wherein assembling and/or self-assembling of the expressed proteins to multi-protein structures takes place in the inner compartment and wherein the expressed proteins remain in the inner fluid until purification and/or isolation, or
wherein assembling and/or self-assembling of the expressed proteins to multi-protein structures takes place in the outer compartment.

4. The method of any of claims 1 - 3,
wherein the inner and the outer compartment are at least partially separated by a membrane and/or wherein the reaction space optionally comprises at least one hollow fiber, and/or
wherein the inner compartment optionally comprises a membrane which differs, preferably by its cut-off value, from the means, being preferably a membrane, separating the inner and outer compartment, and/or
wherein an exchange of predetermined substances occurs across the means separating the outer compartment and the inner compartment, and/or
wherein the exchange of substances is optionally provided by means of dialysis or tangential flow filtration.

5. The method of any of claims 1 - 4,
wherein the outer fluid and the inner fluid are based on the same solution or different solutions, and/or
wherein the outer fluid is directed to flow around or along the inner compartment or is directed to flow through the inner compartment,
and/or wherein the inner fluid and the outer fluid are optionally guided in counterflow, preferably in continuous flow-streams, or non-continuous flow-streams, e.g. sequentially.

6. The method of any of claims 1 - 5,
comprising a step of adding of at least one additive supplementing, supporting and/or modulating protein expression, improving protein stability and/or protein assembly or self-assembly, to the inner compartment and/or to the outer compartment, and/or
wherein said additive is preferably selected from natural or non-natural amino acids, co-factors, tRNAs, rNTPs, dNTPschaperons, polymerases, transcription regulatory factors, and/or any mixtures thereof, and/or
optionally comprises adding at least one additive enhancing protein assembly or self-assembly to the outer compartment, e.g. chaperons, and/or posttranslational protein modification enzymes, such as enzymes and substrates for acylation, alkylation, carboxylation, hydroxylation, methylation, myristoylation, palmitoylation, isoprenylation, lipoylation, glycosylation, etc, and/or
wherein the additives are added continuously or non-continuously.

7. The method of any of claims 1 - 6, wherein the outer fluid is continuously supplied to the inner compartment, e.g. via a continuous supply-stream, or sequentially, e.g. being part of a multi-stage process.

8. The method of any of claims 1 - 7, wherein nucleic acids and/or additives removed from the inner compartment are at least partially reused or recycled.

9. The method of any of claims 1 - 8, which is a replenishing process.

10. The method of any of claims 1-9, wherein the cell-free expression system is host independent and preferably selected from cell lysates or artificial expression systems and wherein the cell lysates are preferably derived from microorganisms, in particular *E. coli,* yeast, insects, mammals, plants and/or are artificial.

11. The method any of claims 1 - 10,
wherein a modified bacteriophage is provided at step (b) by applying at least one step selected of the group:
(i) providing a modified bacteriophage genome,
(ii) adding a modified or unmodified protein to be assembled into the bacteriophage to be provided that differs from the corresponding protein in the corresponding unmodified bacteriophage,
(iv) adding a nucleic acid encoding for a modified or unmodified protein to be assembled into the bacteriophage to be provided, wherein the protein differs from the corresponding protein in the corresponding unmodified bacteriophage,
(v) a nucleic acid molecule that suppresses the expression of a protein of a bacteriophage, and/or
(vi) any combination of steps (i)-(v) thereof.

12. System adapted for the method according to any of claims 1-11,
comprising a reaction space which comprises an inner compartment for taking up an inner fluid and an outer compartment for taking up an outer fluid, which compartments are separated by a means allowing the exchange of predetermined substances.

13. Protein, in particular virus, or bacteriophage, provided by the method of any of claims 1 to 12.

14. An all *in-vitro* method according to any of claims 1 - 12 for the amplification and provision of synthetic bacteriophages.

15. Protein, in particular virus, or bacteriophage according to claim 13 or provided by the method of any of claims 1 - 12 or 13 for use in medicine in particular in a method of treatment, prevention and/or vaccination of a patient in need thereof, as well as in chemistry, biotechnology, agriculture and/or food industry.
